# EUROPÄISCHE PATENTSCHRIFT

(11) **EP 1 935 386 B1**
(45) Veröffentlichungstag und Bekanntmachung des Hinweises auf die Patenterteilung: **20.02.2019**
(21) Anmeldenummer: 06405530.4
(22) Anmeldetag: 19.12.2006
(51) Int. Cl.: A61F 9/06

(54) **Aktives Lichtschutzfilter**
Active light protection filter
Filtre actif contre la lumière

(43) Veröffentlichungstag der Anmeldung: 25.06.2008
(73) Patentinhaber: castelberg design GmbH, 8045 Zürich (CH)
(72) Erfinder: Gunz, Stefan, 8820 Wädenswil (CH); Castelberg, Donata, 8820 Wädenswil (CH); Castelberg, Manfred, 8824 Schönenberg (CH)
(74) Vertreter: Alder, Hans Rudi

(56) Entgegenhaltungen:
- US-A- 5 519 522
- US-A- 5 991 072
- US-A1- 2004 058 094
- IVAR HAMBERG AND CLAES G. GRANQVIST: "Transparent and infrared-reflecting indium-tin-oxide films:quantitative modeling of the optical properties", APPLIED OPTICS, vol. 24, no. 12, 15 June 1985 (1985-06-15) ,

## Beschreibung

Die Erfindung betrifft ein aktives Lichtschutzfilter für Schweissschutzschilder, Schweissschutzbrillen oder dergleichen gemäss Patentanspruch 1.

Die Erfindung betrifft insbesondere ein gattungsgemässes aktives Lichtschutzfilter für Schweissschutzschilder oder Schweissschutzbrillen oder dergleichen, wie zum Beispielauch zum Hartlöten, mit einem bezüglich seiner optischen Transmission elektrisch steuerbaren Filterelement, das mit einer Transmissionssteuereinrichtung in Wirkverbindung steht, wobei das elektrisch steuerbare Filterelement ein Flüssigkristallelement ist. US-A-5'519'552 offenbart ein aktives Lichtschutzfilter für Schweissschutzbrillen.

Ein aktives Schweissschutzfilter ist ein selbst-abdunkelndes Filter auf der Basis von Flüssigkristallzellen, das von einer elektronischen Schaltung gesteuert wird. Es schaltet bei Schweissbeginn blitzschnell in einen stark abgedunkelten Zustand und kehrt bei Schweissende wieder in den Hellzustand zurück. Die Flüssigkristallzelle ist somit das elektrisch steuerbare Filterelement, die elektronische Schaltung die Transmissionssteuereinrichtung. Der Zweck eines solchen Schweissschutzfilters besteht darin, den Schweisser vor übermässiger und gefährlicher Strahlung im sichtbaren Wellenlängenbereich, im Ultraviolettbereich und im Infrarotbereich zu schützen. Die Mindestanforderungen an derartige Filter sind in einschlägigen Normen niedergelegt. Allerdings bestehen für solche Filter bezüglich der Transmissionsanforderungen in bestimmten Wellenlängenbereichen zum Teil signifikante Unterschiede zwischen den Normen der verschiedenen Normengremien wie ANSI (USA) und CE (Europa). Diese Unterschiede betreffen hauptsächlich den Infrarotbereich und das Verhalten von Schweissschutzfiltern im
Infrarotbereich. Letzteres ist auch eines der Hauptthemen der vorliegenden Erfindung.

Abklärungen haben ergeben, dass die heute gültigen Normen für Schweissschutzfilter weitgehend historische Hintergründe haben. Es hat sich herausgestellt, dass die Transmissionsanforderungen einfach von Stahlwerken und Giessereien übernommen wurden. Die europäischen Normen (CE) stellen sehr strenge Anforderungen im Wellenlängenbereich zwischen 780 nm und 1400 nm, die korrespondierenden US-Normen (ANSI) sind zwar etwa um einen Faktor 10 toleranter , verlangen aber Schutz bis 2000 nm. Alle vom Anmelder untersuchten, den Stand der Technik repräsentierenden, aktiven Schweissschutzfilter zeigen aber ein Abfallen des Schutzes über ca. 1500 nm. Die ebenso unerwünschte, noch langwelligere IR-B Strahlung (1400 - 3000 nm) wird nicht gefiltert. Die mit einem Foto-Spektrometer gemessenen Werte zwischen 2000 und 3000 nm waren zum Teil geradezu erschreckend hoch.

Ein weiterer wichtiger Gesichtspunkt ist die Färbung von Schweissschutzfiltern bei der Durchsicht. Alle sich auf dem Markt befindlichen, untersuchten Filter zeigen bei Durchsicht - also in der Anwendung - ein grünes Bild. Dies kann sich in der Praxis als äusserst störend erweisen, denn die Farberkennung kann bei bestimmten Schweisstätigkeiten von grosser Bedeutung sein, nämlich um die Gegenstände im Blickfeld richtig identifizieren und Warnmeldungen, wie beispielsweise rote Leuchtdioden, überhaupt erkennen zu können.

Es hat sich gezeigt, dass beim Stand der Technik mehrheitlich zwei verschiedene Prinzipien zur Anwendung gelangen, deren Transmissionskurven jedoch sehr ähnlich sind. Nahezu alle Produkte sind mit dichroitischen (reflektierenden)Filtern ausgestattet, erkenntlich am magenta-farbenen Farbbeindruck
bei Aufsicht. Diese sind mit Dünnschichttechnologie auf Glas aufgedampft und sind streng grün bei Durchsicht. Einige Filter hatten sogar zusätzlich einen unschönen Gelbstich. Nur ein Hersteller verwendet Kunststoffe, eine Kombination von in PMMA gegossenen IR-Absorbern und einem auf PET aufgedampften Reflektor. Dabei blockt der Absorber kürzerwelliges IR zwischen 780 nm und 1400 nm und der Reflektor senkt den längerwelligen Anteil zwischen 1400 bis 2000 nm ab. Allerdings zeigt auch dieses Produkt bei Durchsicht - also bei der Anwendung - ein grünes Bild der Welt und im Wellenlängenbereich grösser 2000 nm steigt die Transmission ebenfalls signifikant an.

Aufgabe der Erfindung ist es somit, ein verbessertes und ökonomisch günstiges Schweissschutzfilter anzugeben, das auch Schutz vor längerwelliger IR-Strahlung bietet und zudem in der Durchsicht - also bei der Anwendung - ein möglichst farbneutrales Bild gewährt.

Diese Aufgabe wird durch die Merkmale des Patentanspruchs 1 gelöst.

Die Lösung besteht darin, dass bei einem gattungsgemässen Lichtschutzfilter eine Trägerschicht mit einer Filterschicht mit mindestens einer gängigen aufklebbaren farbneutralen strahlungsreflektierenden Folie mit einer filteraktiven Schicht vorhanden ist, die
- im UV-Bereich eine fast völlige Undurchlässigkeit aufweist,
- im sichtbaren Bereich eine fast völlige Transmission aufweist, und
- sowohl im nahen Infrarot-Bereich (NIR) wie auch im kurzwelligen Infrarot-Bereich (SWIR) gegen höhere Wellenlängen eine immer weiter stetig abfallende Transmission aufweist,
wobei die Trägerschicht aus Kunststoff, vorzugsweise aus PET ist und die Filterschicht auf der dem Flüssigkristallelement abgewendeten Seite der Trägerschicht aufgebracht ist.

Filter mit annähernd vergleichbaren Eigenschaften werden beispielsweise in LCD-Projektoren (Beamern) eingesetzt. Ein sehr steiler, farbneutraler Filter schützt die Flüssigkristallzelle vor der starken Hitzeentwicklung der Lichtquelle, meist einer Halogenlampe. Im sichtbaren Bereich sind diese Filter farbneutral, was die Verwendung zum erwähnten Zweck überhaupt erst ermöglicht. Technisch wären solche Filter somit durchaus geeignet, wegen der hohen Preise sind sie kommerziell aber leider unbrauchbar.

Nun gibt es aber auch die heute insbesondere in der Gebäudetechnik bereits vielerorts eingesetzten "Sunscreen"-Folien. Handelsübliche Folien dieser Art haben alle gewünschten Eigenschaften: eine genügende optische Qualität, einen neutral grauen Farbeindruck und insbesondere auch stetig sinkende Transmission im Infrarotbereich. Zudem sind sie auch mit Klebeschichten und Kratzschutz erhältlich und sie sind kommerziell interessant. Sie werden wie erwähnt in grossen Mengen in der Architektur und auch im Automobilbau eingesetzt. Hersteller derartiger Produkte sind beispielsweise Firmen wie Southwall oder 3M und bspw. in US-6'034'813 beschrieben.

Es lässt sich aber zeigen, dass die Transmissionsanforderungen der heutigen Normen - insbesondere des weltweit wichtigen ANSI-Standards 87.1 ohne weiteres erfüllt werden können, insbesondere durch das mehrfache Aufeinanderschichten derartiger Folien in Laminiertechnik.

Es gibt zudem auch berechtigte Gründe zu der Annahme, dass der Wirkung längerwelliger IR-Strahlung und dem Schutz vor längerwelliger IR-Strahlung künftig auch in verbesserten oder zumindest angepassten Normen für Schweissschutzfilter Rechnung getragen wird. Es ist heute nämlich bekannt, dass IR-Strahlung - nicht nur im IR-A-Bereich (auch NIR genannt), also von 780 nm - 1400 nm, sondern auch im IR-B-Bereich (auch SWIR genannt), also von 1400 nm - 3000 nm - insbesondere durch Absorptionswirkungen in den Augen, zu vermehrten Schäden in der Linse, der Iris, der Netzhaut und der Augenflüssigkeit führen kann. Insbesondere der IR-B-Bereich ist in der CE-Norm aber überhaupt nicht berücksichtigt (vgl. dazu die Grafik in der Detailbeschreibung).

Somit stellt das erfindungsgemässe Schweissfilter in mehrfacher Hinsicht eine wesentliche Verbesserung gegenüber dem Stand der Technik bei Schweissschutzfiltern dar.

Im folgenden werden anhand von Zeichnungen die prinzipiellen Anforderungen an Schweissschutzfilter, der Stand der Technik bei Schweissschutzfiltern und ein Ausführungsbeispiel der vorliegenden Erfindung näher erläutert.

In den Zeichnungen zeigen:
- Fig. 1: eine schematische Darstellung der Transmissionskurve eines "idealen" Schweissschutzfilters,
- Fig. 2: eine schematische Darstellung der "Sperrbereiche" gemäss den geltenden Normen (CE und ANSI),
- Fig. 3: eine schematische Darstellung des Schichtaufbaus eines gängigen Schweissschutzfilters gemäss dem Stand der Technik,
- Fig. 4: den prinzipiellen Verlauf der Transmissionskurve eines Schweissschutzfilters gemäss Figur 3,
- Fig. 5: eine schematische Darstellung des Schichtaufbaus eines erfindungsgemässen Schweissschutzfilters,
- Fig. 6: den prinzipiellen Verlauf der Transmissionskurve einer Filterschicht für ein Schweissschutzfilter gemäss Figur 5,
- Fig. 7: ein Blockschema einer Transmissionssteuereinrichtung für ein erfindungsgemässes Schweissschutzfilter gemäss Fig. 5.

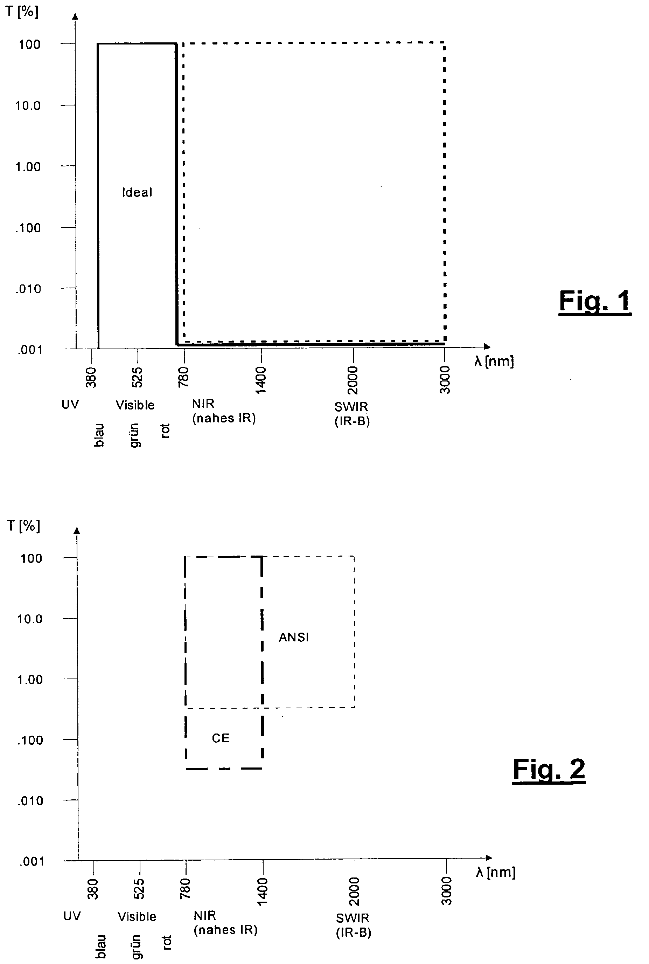

Die Figur 1 zeigt eine schematische Darstellung der Transmissionskurve eines "idealen" Schweissschutzfilters. In der Praxis ist das natürlich nicht oder nur annäherungsweise realisierbar. Ein ideales Filter hat eine absolute Strahlungsundurchlässigkeit im UV-Bereich, also für Wellenlängen kleiner 390 nm; ferner eine (im nichtabgedunkelten Zustand) völlige Durchlässigkeit im sichtbaren Bereich (390 nm bis 780 nm) und weiterhin eine wiederum absolute Strahlungsundurchlässigkeit im IR-Bereich, also für Wellenlängen grösser 780 nm. Üblicherweise wird im Bereich der infraroten Strahlung der Wellenlängenbereich zwischen 780 nm - 1400 nm als IR-A-Bereich bezeichnet (auch NIR = Near Infrared genannt); der Wellenlängenbereich zwischen 1400 nm - 3000 nm als IR-B-Bereich bezeichnet (auch SWIR = Short Wavelength IR genannt).

Die Figur 2 zeigt eine schematische Darstellung der "Sperrbereiche" gemäss den geltenden Normen (CE und ANSI). Die Europäischen Norme (CE) stellt sehr strenge Anforderungen im Wellenlängenbereich zwischen 780 nm und 1400 nm, die korrespondierenden US-Normen (ANSI) sind etwa um einen Faktor 10 toleranter, spezifizieren dafür aber Schutz bis 2000 nm. So ist aus der Figur 2 erkennbar, dass die maximale Transmission (T) eines Schweissschutzfilters der Schutzstufe 11 im IR-A Bereich gemäss CE-Norm höchstens 0,05 % betragen darf. Gemäss ANSI ist in diesem Bereich jedoch ein Grenzwert von 0,5 % zulässig.

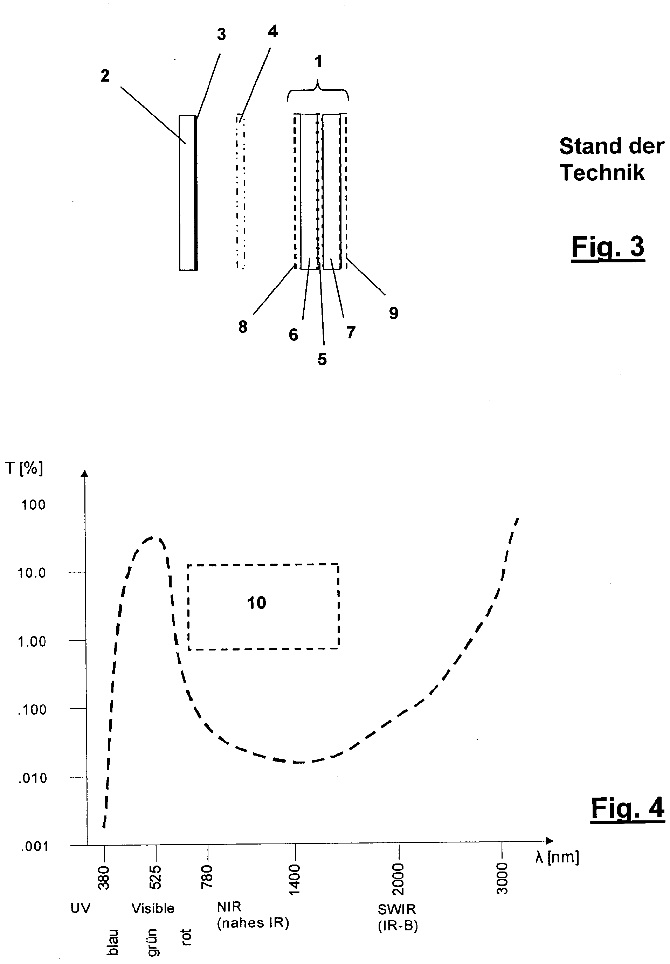

Die Figur 3 zeigt eine schematische Darstellung des Schichtaufbaus eines gängigen Schweissschutzfilters gemäss dem Stand der Technik. Ein derartiges Schweissschutzfilter enthält ein elektrisch steuerbares Filterelement in der Form eines Flüssigkristallelementes 1. Weiterhin enthält das Schweissschutzfilter eine Trägerschicht 2 mit einer auf der Trägerschicht 2 aufgebrachten Filterschicht 3. Die Trägerschicht 2 ist zumeist aus Glas und ist auf das Flüssigkristallelement 1 aufgeklebt, angedeutet durch die Klebschicht 4. Die Filterschicht 3 ist üblicherweise ein dichroitisches Filter das mit gängiger Dünnschichttechnologie auf die Trägerschicht 2 aus Glas aufgedampft ist. Je nach Aufbau des Schweissschutzfilters kann sich die Filterschicht 3 auf der einen oder anderen Seite der Trägerschicht befinden. Weitere (nicht dargestellte) Schichten, wie beispielsweise Kratzschutzschichten, können auch noch vorhanden sein.

Das Flüssigkristallelement 1 ist hier in einem ebenfalls sehr häufig verwendeten Aufbau gezeigt. Eine Flüssigkristallschicht 5 befindet sich zwischen zwei leitend beschichteten Glasplatten 6, 7. Die leitenden Beschichtungen der Glasplatten 6, 7 sind ein Front-Polarisator 8 und ein Rück-Polarisator 9. Das Flüssigkristallelement wird hier im sogenannt symmetrisch-transmissiven Betrieb verwendet. Die zur Ansteuerung des Flüssigkristallelements notwendige elektronische Transmissionssteuereinrichtung ist hier zwecks Vereinfachung der Darstellung aber nicht gezeigt. Es besteht grundsätzlich auch eine Notwendigkeit, ein Flüssigkristallelement mit genau diesem Aufbau und genau dieser Funktionsweise einzusetzen. Da muss lediglich gewährleistet sein, dass sich die Flüssigkristallschicht mittels der Transmissionssteuereinrichtung zwischen hell (transparent) und dunkel (beinahe intransparent) mit sehr kurzer Reaktionszeit umschalten lässt.

Die Figur 4 zeigt noch den prinzipiellen Verlauf der Transmissionskurve gemäss dem gängigen Stand der Technik, wie bspw. aus der US-6'565'982 bekannt. Der dargestellte Verlauf zeigt den qualitativen Charakter der Transmissionskurve. Da dichroitische Filter bekannterweise sehr schmalbandige steile Filter sind, ist der steile Anstieg und Abfall im sichtbaren Bereich des Spektrums deutlich erkennbar. Ausserhalb des sichtbaren Spektrums, d.h. mit zunehmender Wellenlänge im infraroten Bereich des Spektrums nimmt die Transmission allmählich wieder zu und steigt zum Teil auf sehr hohe Werte an. In einem angedeuteten Bereich 10 befinden sich die "IR-Sperrbereiche" gemäss den eizuhaltenden Normen (vgl. dazu Fig. 2).

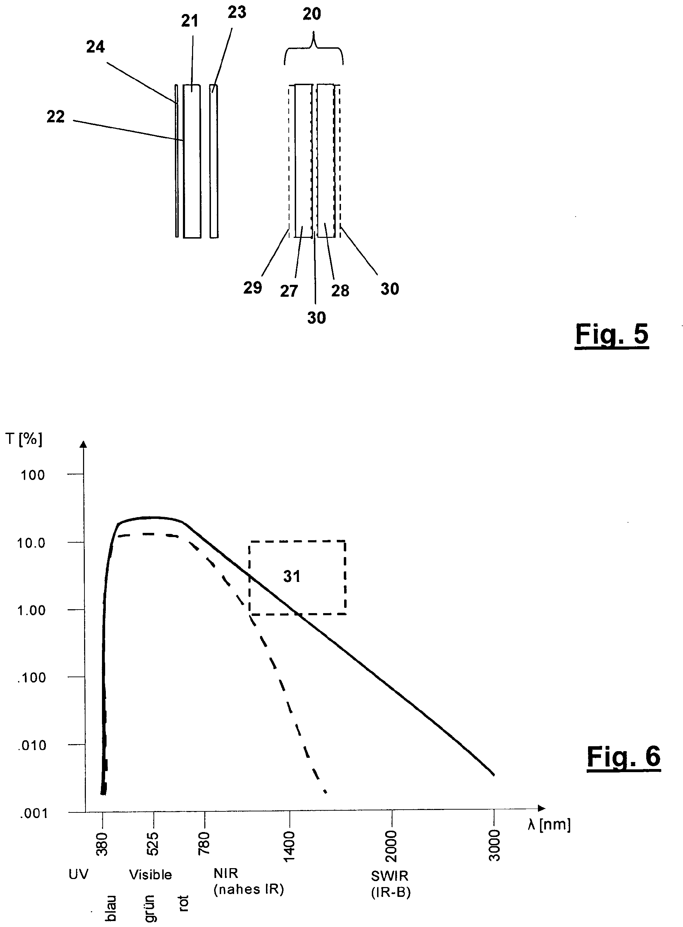

Die Figuren 5 - 7 zeigen nunmehr ein mögliches Ausführungsbeispiel der vorliegenden Erfindung.

Die Figur 5 zeigt eine Schematische Darstellung des Schichtaufbaus eines erfindungsgemässen Schweissschutzfilters. Ein erfindungsgemässes Schweissschutzfilter enthält ein elektrisch steuerbares Filterelement in der Form eines Flüssigkristallelements 20. Weiterhin enthält das Schweissschutzfilter eine Trägerschicht 21 mit einer auf der Trägerschicht 21 aufgebrachten Filterschicht 22. Die Filterschicht 22 umfasst mindestens eine filteraktive Schicht mit den nachstehend genannten Eigenschaften bezüglich Transmission im relevanten Strahlungsbereich. Die Trägerschicht 21 ist aus Kunststoff und ist auf das Flüssigkristallelement 20 aufgeklebt, angedeutet durch die Klebschicht 23. Die Trägerschicht 21 und die Filterschicht 22 sind vorzugsweise zusammen als aufgeklebte sogenannte "Sunscreen"-Folie realisiert, es kann aber auch (herstellungsmässig) eine separate Trägerschicht mit darauf aufgeklebter Sunscreen-Folie vorhanden sein. Weitere Schichten, wie beispielsweise eine Kratzschutzschicht 24, können auch noch vorhanden sein. Ebenso ist es möglich, dass die Trägerschicht 21, die Filterschicht 22, der Kratzschutz 24 und auch die Klebschicht 23 in einer einzigen Folie oder Schicht zusammengefasst sind. Handelsübliche Sunscreen-Folien dieser Art sind im wesentlichen aufklebbare farbneutrale strahlungsreflektierende Filter (d.h. strahlungsreflektierend für die unerwünschten Wellenlängenbereiche); sie haben jedoch nicht die extreme Flankensteilheit dichroitischer Filter und sie sind deshalb auch viel kostengünstiger. Dennoch besitzen sie eine gute optische Qualität im sichtbaren Bereich, da sie ja auch für Fensterbeschichtungen verwendet werden. Sie vermitteln insbesondere bei weissem Durchlicht einen annähernd neutral grauen Farbeindruck und sie weisen im Infrarotbereich gegen höhere Wellenlängen eine stetig sinkende Transmission auf. Zudem sind diese Folien auch im UV-Bereich fast völlig undurchsichtig, was im Bereich des Schweissens natürlich ebenfalls von sehr grosser Bedeutung ist, da insbesondere Schweisslichtbogen auch sehr starke UV-Strahler sind. Je nach Aufbau des Schweissschutzfilters kann sich die mindestens eine Filterschicht 22 natürlich auch hier auf der einen oder anderen Seite der Trägerschicht 21 befinden.

Die Trägerschicht 21 besteht aus einem Kunststoff, beispielsweise aus PET. Vorzugsweise werden sogenannte "gereckte" PET-Folien verwendet, da diese in der Summe der Anforderungen für grossflächiges Aufbringen auf Glas (gute optische Eigenschaften, hohe Zugfestigkeit, tiefer linearer thermischer Ausdehnungskoeffizient) besonders gut geeignet sind. Es können aber auch andere optisch geeignete Kunststoffe verwendet werden. Beigegebene Additive schützen die Trägerschicht 21 gegen das Aufspalten der Kohlenstoffverbindungen des Kunststoffes durch ultraviolette Strahlung. Ohne diese Massnahme würden die Folien bei den starken UV-Strahlungen, die beim Lichtbogenschweissen entstehen, rasch trüb und bräunlich und somit unbrauchbar.

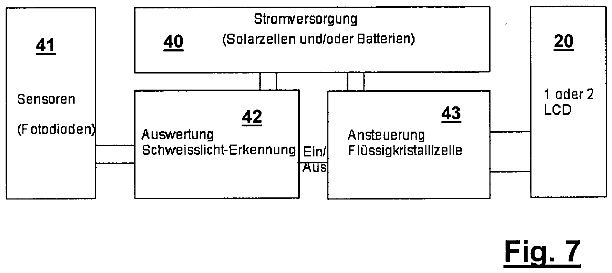

Das Flüssigkristallelement 20 kann im gleichen häufig verwendeten Aufbau wie beim Stand der Technik gemäss Fig. 3 verwendet werden. Eine Flüssigkristallschicht 26 befindet sich zwischen zwei leitend beschichteten Glasplatten 27, 28. Die leitenden Beschichtungen der Glasplatten 27, 28 sind ein Front-Polarisator 29 und ein Rück-Polarisator 30. Das Flüssigkristallelement wird hier im sogenannt positivtransmissiven Betrieb verwendet. Die Ansteuerung erfolgt derart, dass bei anliegender Spannung an den Polarisatoren das Flüssigkristallelement 20 auf ,Dunkel' geschaltet wird. Es können grundsätzlich aber auch mehrere Flüssigkristallelemente vorgesehen sein, beispielsweise zwei oder drei. Üblicherweise werden Flüssigkristallelemente vom Typ TN (Twisted Nematic) eingesetzt, es können aber auch deren Weiterentwicklungen STN oder DSTN oder auch andere Typen, beispielsweise solche mit nur einem Polarisator, sein. Die zur Ansteuerung des Flüssigkristallelements notwendige elektronische Transmissionssteuereinrichtung ist auch hier zwecks Vereinfachung der Darstellung nicht gezeigt (siehe dazu aber Fig. 7). Wie erwähnt besteht grundsätzlich keine Notwendigkeit, ein Flüssigkristallelement mit genau dem in Fig. 5 gezeigten Aufbau einzusetzen. Es muss lediglich gewährleistet sein, dass sich die Flüssigkristallschicht mittels der Transmissionssteuereinrichtung zwischen hell (transparent) und dunkel (beinahe intransparent) mit sehr kurzer Reaktionszeit umschalten lässt.

Es besteht nun aber natürlich die Möglichkeit, mehrere der genannten Sunscreen-Folien übereinander zu legen und so insgesamt eine Filtercharakteristik mit viel grösserer Flankensteilheit zu erhalten. Es hat sich gezeigt, dass mit den heute erhältlichen Sunscreen-Folien - beispielsweise solchen von Southwall - vier derart übereinandergelegte strahlungsreflektierende Folien genügen, um eine derartige Flankensteilheit zu erzielen, dass die ANSI-Normen bezüglich Schweissschutzfiltern eingehalten werden können. Zudem ergibt sich der Vorteil, dass die resultierende Filtercharakteristik bezüglich IR-Strahlung gegen höhere Wellenlängen sogar noch eine verstärkt stetig abfallende Transmission aufweist. Ein Nachteil ist lediglich insofern in Kauf zu nehmen, als durch das Übereinanderlegen mehrerer Sunscreen-Folien auch im sichtbaren Bereich eine Reduktion der Transmission erfolgt. Mit einer Folie erreicht man heute im sichtbaren Bereich Transmissionswerte von 70 % - 75 %. Mit vier übereinanderliegenden Folien sind es dann noch Werte von ca. 30 %. Die annähernd neutrale Farbwiedergabe bei Durchsicht bleibt aber erhalten.

Weitere Verbesserungen der Eigenschaften von Sunscreen-Folien können zudem dazu führen, dass die Zahl der benötigten Folien künftig reduziert werden kann. Dies wäre insbesondere dann der Fall, wenn das Reflexionsverhalten der Sunscreen-Folien im IR-B Bereich noch weiter verbessert wird.

Die Figur 6 zeigt den prinzipiellen Verlauf der Transmissionskurve einer Filterschicht 22 für ein Schweissschutzfilter gemäss Figur 5. Sie zeigt den qualitativen Verlauf der Gesamttransmissionskurve eines erfindungsgemässen Schweissschutzfilters mit einer einzigen Sunscreen-Folie.

Deutlich sichtbar ist die gegen höhere Wellenlängen im IR-Bereich immer weiter stetig abfallende Transmission. Mit zunehmender Anzahl der verwendeten Sunscreen-Folien wird die Flankensteilheit der Gesamtkurve in diesem Bereich immer höher (angedeutet durch die gestrichelte, steilere Linie). Auf diese Weise kann dafür gesorgt werden, dass die Flankensteilheit eines erfindungsgemässen Schweissschutzfilters im IR-A-Bereich hoch genug ist, um die in einem (hier lediglich angedeuteten) Bereich 31 befindlichen "IR-Sperrbereiche" zu vermeiden und so die einschlägigen Normen einzuhalten (vgl. dazu auch Fig. 2).

Die Figur 7 zeigt noch ein Blockschema einer Transmissionssteuereinrichtung für ein erfindungsgemässes selbst-abdunkelndes (aktives) Schweissschutzfilter gemäss Fig. 5. Der prinzipielle Aufbau ist einfach. Neben einer Stromversorgung 40 in der Form von Solarzellen oder Batterien braucht es einen Sensor 41, eine Auswerteschaltung 42 und eine Ansteuerungsschaltung 43 für die Ansteuerung des Flüssigkristallelementes 20. Der Sensor 41 ist üblicherweise mindestens eine Fotodiode zur Erkennung der Lichtemission des Schweisslichtbogens. Die Auswerteschaltung 42 dient zur Auswertung des Signales von der Fotodiode und erzeugt ein Ein/Aus-Steuersignal für das Flüssigkristallelement 20. Die Ansteuerschaltung 43 übernimmt das Ein/Aus-Steuersignal von der Auswerteschaltung 42 und schaltet das Flüssigkristallelement 20 in den durchsichtigen oder undurchsichtigen Zustand.

Viele der gängigen aktiven Schweissschutzfilter benötigen eine Batterie zur Speisung der Transmissionssteuereinrichtung. Es gibt aber auch Schweissschutzfilter mit eingebauten Solarzellen die ohne Backup-Batterien auskommen und die selbst bei relativ ungünstigen Lichtverhältnissen, d.h. bei relativ geringen Beleuchtungsstärken, zuverlässig arbeiten. Dennoch ist die zur Verfügung stehende Energie natürlich immer relativ knapp, so dass diesbezüglich weitere Verbesserungen wünschbar sind.

In einer vorteilhaften Weiterentwicklung des erfindungsgemässen Schweissschutzfilters kann somit die Stromversorgung der Transmissionssteuereinrichtung auch eine transparente Solarzelle sein, die im Schichtaufbau des Lichtschutzfilters integriert ist. Da es unterdessen bereits marktreife transparente Solarzellen gibt, beispielsweise solche von Sunways, können derartige Produkte auch in moderne Schweissschutzfilter integriert werden. Mit einer derartigen Lösung kann sichergestellt werden, dass immer genug elektrische Energie zur Verfügung steht um die notwendige Betriebssicherheit zu gewährleisten.

### Bezugsziffernliste:

zum Stand der Technik:
- 1: Flüssigkristallelement
- 2: Trägerschicht
- 3: Filterschicht
- 4: Klebschicht
- 5: Flüssigkristallschicht
- 6: Glasplatte
- 7: Glasplatte
- 8: Front-Polarisator
- 9: Rück-Polarisator
- 10: Bereich (der IR-Sperrzonen)
- 11-19: nicht verwendet

zur Erfindung:
- 20: Flüssigkristallelement
- 21: Trägerschicht
- 22: Filterschicht
- 23: Klebschicht
- 24: Kratzschutzschicht
- 25: nicht verwendet
- 26: Flüssigkristallschicht
- 27: Glasplatte
- 28: Glasplatte
- 29: Front-Polarisator
- 30: Rück-Polarisator
- 31: Bereich (der IR-Sperrzonen)
- 32-39: nicht verwendet
- 40: Stromversorgung
- 41: Sensor
- 42: Auswerteschaltung
- 43: Ansteuerschaltung

## Patentansprüche

1. Aktives Lichtschutzfilter für Schweissschutzschilder, Schweissschutzbrillen oder dergleichen, mit einem bezüglich seiner optischen Transmission elektrisch steuerbaren Filterelement, das mit einer Transmissionssteuereinrichtung in Wirkverbindung steht, wobei das elektrisch steuerbare Filterelement ein Flüssigkristallelement (20) ist und auf dem Flüssigkristallelement (20) eine Trägerschicht aufgeklebt (21) ist und wobei auf der Trägerschicht (21) eine Filterschicht (22) aufgebracht ist, **dadurch gekennzeichnet, dass** die Filterschicht (22) eine filteraktive Schicht enthält, die mehrere aufeinandergeschichtete und für Fensterbeschichtungen gängige, aufklebbare, farbneutrale, strahlungsreflektierende "Sunscreen"-Folien umfasst, die
- im UV-Bereich eine fast völlige Undurchlässigkeit aufweisen,
- im sichtbaren Bereich eine fast völlige Transmission aufweisen, und
- sowohl im nahen Infrarot-Bereich (IR-A) wie auch im kurzwelligen Infrarot-Bereich (IR-B) gegen höhere Wellenlängen eine immer weiter stetig abfallende Transmission aufweisen,
wobei die Trägerschicht (21) aus Kunststoff, vorzugsweise aus PET ist und die Filterschicht (22) auf der dem Flüssigkristallelement (20) abgewendeten Seite der Trägerschicht (21) aufgebracht ist.

2. Aktives Lichtschutzfilter nach Patentanspruch 1, **dadurch gekennzeichnet, dass** die fast völlige Transmission der filteraktiven Schicht im sichtbaren Bereich darin besteht, dass die Filterschicht (22) bei weissem Durchlicht eine annähernd graue Färbung aufweist.

3. Aktives Lichtschutzfilter nach Patentanspruch 1 oder 2, **dadurch gekennzeichnet, dass** die Filterschicht (22) mehrere, vorzugsweise vier übereinandergeklebte gleichartige strahlungsreflektierende Folien umfasst.

4. Aktives Lichtschutzfilter nach einem der Patentansprüche 1 bis 3, **dadurch gekennzeichnet, dass** über der Filterschicht (22) eine Kratzschutzschicht (24) vorhanden ist.

5. Aktives Lichtschutzfilter nach einem der Patentansprüche 1 bis 4, **dadurch gekennzeichnet, dass** die Stromversorgung der Transmissionssteuereinrichtung eine Solarzelle und/oder Batterien sind.

6. Aktives Lichtschutzfilter nach Patentanspruch 5, **dadurch gekennzeichnet, dass** die Stromversorgung der Transmissionssteuereinrichtung eine transparente Solarzelle ist, die im Schichtaufbau des Lichtschutzfilters integriert ist.

## Claims

1. Active light protection filter for welding shields, protective welding goggles or the like, having a filter element that can be electrically controlled with regard to the optical transmission thereof, wherein the filter element has an operative connection to the transmission control unit, the filter element that can be controlled being a liquid crystal element (20) with a carrier layer (21) glued to the liquid crystal element (20) and a filter layer (22) applied to the carrier layer (21), **characterized in that** the filter layer (22) comprises a filter-active layer, consisting of several "sunscreen" films bonded over one another which as usual for widow-coatings are radiation-reflecting, adhesive and colour-neutral,
- having nearly complete impermeability in the UV range,
- nearly complete transmission in the visible range, and
- continuously decreasing transmission both in the near infrared range (IR-A) as well as in the short-wave infrared range (IR-B) against greater wavelengths, the carrier layer (21) being made of plastic, preferably PET and the filter layer (22) being applied on the side of the carrier layer (21) turned away from the liquid crystal element (20).

2. Active light protection filter according to patent claim 1, **characterized in that** the nearly complete transmission of the filter-active layer in the visible range is due to the fact that the filter layer (22) has an approximately grey coloration in the case of white transmitted light.

3. Active light protection filter according to patent claim 1 or 2, **characterized in that** the filter layer (22) comprises several, preferably four, adhesive, identical and radiation-reflecting films bonded over one another.

4. Active light protection filter according to one of patent claims 1 to 3, **characterized in that** a scratch-resistant layer (24) is present over the filter layer (22).

5. Active light protection filter according to one of patent claims 1 to 4, **characterized in that** the power supply to the transmission control unit is a solar cell and/or batteries.

6. Active light protection filter according to patent claim 5, **characterized in that** the power supply to the transmission control unit is a transparent solar cell, which is integrated in the layer structure of the light protection filter.

## Revendications

1. Filtre actif de protection contre la lumière pour boucliers de protection de soudage, lunettes de protection de soudage ou similaires, avec un élément filtrant pouvant être commandé électriquement quant à sa transmission optique, qui est en liaison active avec un dispositif de commande de transmission, dans lequel l'élément filtrant pouvant être commandé électriquement est un élément à cristaux liquides (20) et une couche de support est collée (21) sur l'élément à cristaux liquides (20) et dans lequel une couche filtrante (22) est appliquée sur la couche de support (21), **caractérisé en ce que** la couche filtrante (22) contient une couche à filtre actif, qui comprend plusieurs feuilles « Sunscreen » réfléchissant le rayonnement, de couleur neutre, adhésives, courantes pour les revêtements de fenêtre et superposées, qui
- présentent une opacité quasi-totale dans le domaine des UV,
- présentent une transmission quasi-totale dans le domaine visible, et
- présentent aussi bien dans le domaine infrarouge proche (IR-A) que dans le domaine infrarouge à ondes courtes (IR-B) une transmission décroissant de plus en plus de façon continue avec l'augmentation des longueurs d'ondes,
dans lequel la couche de support (21) est en matière plastique, de préférence en PET et la couche filtrante (22) est appliquée sur le côté de la couche de support (21) opposé à l'élément à cristaux liquides (20).

2. Filtre actif de protection contre la lumière selon la revendication 1, **caractérisé en ce que** la transmission quasi-totale de la couche à filtre actif dans le domaine visible consiste dans le fait que la couche filtrante (22) présente une teinte approximativement grise en cas de lumière transmise blanche.

3. Filtre actif de protection contre la lumière selon la revendication 1 ou 2, **caractérisé en ce que** la couche filtrante (22) comprend plusieurs, de préférence quatre feuilles réfléchissant le rayonnement, de même type, collées les unes sur les autres.

4. Filtre actif de protection contre la lumière selon l'une quelconque des revendications 1 à 3, **caractérisé en ce qu'**une couche anti-rayure (24) est présente au-dessus de la couche filtrante (22).

5. Filtre actif de protection contre la lumière selon l'une quelconque des revendications 1 à 4, **caractérisé en ce que** l'alimentation électrique du dispositif de commande de transmission est une cellule solaire et/ou des batteries.

6. Filtre actif de protection contre la lumière selon la revendication 5, **caractérisé en ce que** l'alimentation électrique du dispositif de commande de transmission est une cellule solaire transparente qui est intégrée dans la structure en couches du filtre de protection contre la lumière.
